# EUROPEAN PATENT APPLICATION

(11) **EP 1 004 561 A1**
(43) Date of publication of application: **31.05.2000**
(21) Application number: 98204025.5
(22) Date of filing: 27.11.1998
(51) Int. Cl.: C07C 1/04

(54) **Process for the production of liquid hydrocarbons**

(71) Applicant: SHELL INTERNATIONALE RESEARCH MAATSCHAPPIJ B.V., 2596 HR Den Haag (NL)
(72) Inventor: Engel, Dirk Coenraad, 1031 CM Amsterdam (NL); Senden, Mathijs Maria Gerardus, 2596 HR The hague (NL); Van der Zwet, Gerardus Petrus, 1031 CM Amsterdam (NL)

(57) **Abstract**

Process for producing normally liquid hydrocarbons from a hydrocarbonaceous feed which process comprises the following steps:
(i) partial oxidation of the hydrocarbonaceous feed at elevated temperature and pressure using an oxygen containing gas to obtain synthesis gas;
(ii) catalytically converting at least part of the synthesis gas mixture obtained in step (i) at elevated temperature and pressure into normally liquid hydrocarbons, normally gaseous hydrocarbons, optionally heavy wax and water;
(iii) expanding and/or combusting at least part of the normally gaseous hydrocarbons produced in step (ii) to provide power for compressing the hydrocarbonaceous feed used in step (i), the gaseous hydrocarbons containing any unconverted synthesis gas and/or hydrocarbonaceous feed.
The hydrocarbonaceous feed is especially associated gas.

## Description

The present invention relates to a further improvement in the optimisation of the production of liquid hydrocarbons from a hydrocarbonaceous feed, especially the optimisation of an integrated, low-cost process for the production of normally liquid hydrocarbons from natural gas, especially associated gas at remote locations as well as at offshore platforms.

Many publications are known describing processes for the conversion of (gaseous) hydrocarbonaceous feed stocks, as methane, natural gas and/or associated gas, into liquid products, especially methanol and liquid hydrocarbons, particularly paraffinic hydrocarbons. In this respect often reference is made to remote locations (e.g. in the dessert, tropical rain-forest) and/or offshore locations, where no direct use of the gas is possible, usually due to the absence of large populations and/or the absence of any industry. Transportation of the gas, e.g. through a pipeline or in the form of liquefied natural gas, requires extremely high capital expenditure or is simply not practical. This holds even more in the case of relatively small gas production rates and/or fields. Reinjection of gas will add to the costs of oil production, and may, in the case of associated gas, result in undesired effects on the crude oil production. Burning of associated gas has become an undesired option in view of depletion of hydrocarbon sources and air pollution. Gas found together with crude oil is known as associated gas, whereas gas found separate from crude oil is known as non-associated gas. Associated gas may be found as "solution gas" dissolved within the crude oil, and/or as "gas cap gas" adjacent to the main layer of crude oil. Associated gas is usually much richer in the larger hydrocarbon molecules (ethane, propane, butane) than non-associated gas.

In WO 94/21512 a process for the production of methanol has been described from an offshore natural gas field using a floating platform. However, no optimally integrated, efficient, low-cost process scheme has been described.

In WO 97/12118 a method and system for the treatment of a well stream from an offshore oil and gas field has been described. Natural gas is converted into syngas using pure oxygen in an autothermal reformer, a combination of partial oxidation and adiabatic steam reforming. The syngas (comprising a considerable amount of carbon dioxide) is converted into liquid hydrocarbons and wax. No fully and optimally integrated process scheme for a highly efficient, low capital process is described in this document.

In WO 91/15446 a process is described to convert natural gas, particularly remote location natural gas (including associated gas), in the form of normally liquid hydrocarbons suitable for fuel use via methanol/dimethyl ether. However, no optimally integrated, efficient, low-cost process scheme has been described.

In US 4,833,170 a process is described for the production of heavier hydrocarbons from one or more gaseous light hydrocarbons. The light hydrocarbons are converted into syngas by autothermal reforming with air in the presence of recycled carbon dioxide and steam. However, no fully optimised and (energy) integrated, efficient, low-cost process scheme has been described.

An object of the present invention is to provide further features for efficient, low cost, compact, process- and energy-integrated process schemes for the production of (easily manageable) normally liquid hydrocarbons from light hydrocarbons, especially by further energy integration. The invention especially relates to the compression of the hydrocarbonaceous feed using energy generated by expanding/combustion gaseous hydrocarbons produced in the hydrocarbon synthesis step, optionally in combination with any non-converted hydrocarbonaceous feed and/or synthesis gas. This is especially important in the case of associated gas, which, after separation from the crude oil, is usually available at low pressure or even at ambient pressure only. Another important application is formed by low pressure gas fields or largely depleted gas fields, having only a low remaining pressure. In a preferred embodiment, in which catalytic partial oxidation is used, especially with oxygen enriched air made by using membrane technology, mainly normally liquid paraffins are produced in a once through, (proven) fixed bed, a very efficient, low capital cost process is provided, which process can be carried out in a compact, relatively light weight plant, making it very suitable for use on a platform or a barge, or in a dismountable plant.

The present invention therefore relates to a process for producing normally liquid hydrocarbons from a hydrocarbonaceous feed which process comprises the following steps:
(i) partial oxidation of the hydrocarbonaceous feed at elevated temperature and pressure using an oxygen containing gas to obtain synthesis gas;
(ii) catalytically converting at least part of the synthesis gas mixture obtained in step (i) at elevated temperature and pressure into normally liquid hydrocarbons, normally gaseous hydrocarbons, optionally heavy wax and water;
(iii) expanding and/or combusting at least part of the normally gaseous hydrocarbons produced in step (ii) to provide power for compressing the hydro-carbonaceous feed used in step (i), the gaseous hydrocarbons containing any unconverted synthesis gas and/or hydrocarbonaceous feed.

A major advantage of the present invention is that relatively simple and cheap processes and apparatus can be used to convert light hydrocarbons into mainly normally liquid hydrocarbons, and if desired energy. Further an optimal use of feedstock and energy is obtained. In a preferred embodiment an optimum carbon conversion (gas into syncrude, minimal carbon dioxide emission), is obtained. In addition, by producing normally liquid hydrocarbons, these hydrocarbons may be mixed with crude oil and transported together.

The hydrocarbonaceous feed suitably is methane, natural gas, associated gas or a mixture of C₁₋₄ hydrocarbons, preferably associated gas. The feed comprises mainly, i.e. more than 80 v/v%, especially more than 92%, C₁₋₄ hydrocarbons, especially comprises at least 60 v/v percent methane, preferably at least 75 percent, more preferably 90 percent. Very suitably natural gas or associated gas is used, especially associated gas at a remote location or an offshore location. Suitably, any sulphur in the feedstock is removed in an absorption tower, comprising e.g. iron and/or zinc oxide. In some cases the natural or associated gas comprises carbon dioxide and/or nitrogen, e.g. in amounts up to 15 or even 25 v/v percent of each of these compounds on the total feed.

The normally liquid hydrocarbons mentioned in the present description are suitably C₄₋₂₄ hydrocarbons, especially C₅₋₂₀ hydrocarbons, more especially C₆₋₁₆ hydrocarbons, or mixtures thereof. These hydrocarbons or mixtures thereof are liquid at temperatures between 5 and 30 °C (1 bar), especially at 20 °C (1 bar), and usually are paraffinic of nature, although considerable amounts of olefins and/or oxygenates may be present. Suitably up to 20 wt%, preferably up to 10 wt%, of either olefins or oxygenated compounds may be present. The heavy wax comprises all hydrocarbons or mixtures thereof which are solid at 20 °C, especially C₁₈₋₂₀₀, more especially C₂₀₋₁₀₀.

The partial oxidation of gaseous feedstocks, producing mixtures of especially carbon monoxide and hydrogen, can take place according to various established processes. These processes include the Shell Gasification Process. A comprehensive survey of this process can be found in the Oil and Gas Journal, September 6, 1971, pp 86-90. The reaction is suitably carried out at temperatures between 800 and 2000 °C and pressures between 4 and 80 bara.

Suitable processes for catalytic partial oxidation have been described in the literature. Very suitable processes for partial oxidation are catalytic partial oxidation processes, especially as described in the European patent applications 576 096, 629 578, 645 344, 656 317 and 773 906. Suitable bed structures are monolith structures, especially ceramic foams, but also metal based metal structures may be used. The monolithic structures may comprise inorganic materials of high temperature resistance, selected from compounds of elements of Groups IIa, IIIa, IVa, IIIb, IVb and the lanthanide group of the Periodic Table of the Elements. Preferably the monolithic structure is zirconia based, especially stabilised zirconia. Suitable active metals for the CPO process rhodium, platinum, palladium osmium, iridium, and ruthenium, preferably rhodium and iridium.

The partial oxidation temperature is usually between 700 and 1300 °C, suitably between 800 and 1200 °C, preferably between 850 and 1050 °C, and the pressure is usually between 4 and 80 bar absolute, suitably between 10 and 50 bar absolute, preferably between 20 and 40 bar absolute. The gas hourly space velocity is suitably in the range of 50,000 to 100,000,000 Nl/l/h (Nl defined as STP, 0 °C, 1 bar), preferably 500,000 to 50,000,000 Nl/l/h, especially 1,000,000 to 20,000,000 Nl/l/h.

The oxygen containing gas may be air (containing about 21 percent of oxygen), oxygen enriched air or pure oxygen. Oxygen enriched air suitably contains up to 70 percent of oxygen, preferably contains up to 60 volume percent, more preferably between 30 and 40 percent of oxygen. Oxygen enriched air and pure oxygen may be produced via cryogenic techniques, but is preferably produced by a membrane based process, e.g. the process as described in WO 93/06041.

To adjust the H₂/CO ratio in the syngas, carbon dioxide and/or steam may be introduced into the partial oxidation process. Preferably up to 15% volume based on the amount of syngas, preferably up to 8% volume, more preferable up to 4% volume, of either carbon dioxide or steam is added to the feed. As a suitable steam source, water produced in the hydrocarbon synthesis may be used. As a suitable carbon dioxide source, carbon dioxide from the effluent gasses of the expanding/combustion step may be used. The H₂/CO ratio of the syngas is suitably between 1.5 and 2.3, preferably between 1.8 and 2.1. If desired, (small) additional amounts of hydrogen may be made by steam methane reforming, preferably in combination with the water shift reaction. Any carbon monoxide and carbon dioxide produced together with the hydrogen may be used in the hydrocarbon synthesis reaction or recycled to increase the carbon efficiency. To keep the process as simple as possible, additional hydrogen manufacture will usually not be a preferred option.

In another embodiment the H₂/CO ratio of the syngas obtained in the catalytic oxidation step may be decreased by removal of hydrogen from the syngas. This can be done by conventional techniques, as pressure swing adsorption or cryogenic processes. A preferred option is a separation based on membrane technology. In the case that hydrogen is removed from the syngas it might be preferred to use a two-stage Fischer-Tropsch process. The hydrogen is than mixed with the gaseous products of the first stage, and together introduced in the second stage. The C₅+ selectivity can be improved in this line-up. Part of the hydrogen may be used in a hydrocracking step of especially the heaviest hydrocarbon fraction of the Fischer-Tropsch reaction.

The percentage of hydrocarbonaceous feed which is converted in the first step of the process of the invention is suitably 50-99% by weight and preferably 80-98% by weight, more preferably 85-96% by weight.

The synthesis gas obtained in the first step is cooled to a temperature between 100 and 500 °C, suitably between 150 and 450 °C, preferably between 300 and 400 °C, preferably under the simultaneous generation of power, e.g. in the form of steam. Further cooling to temperatures between 40 and 130 °C, preferably between 50 and 100 °C, is done in a conventional heat exchanger, especially a tubular heat exchanger. To remove any impurities from the syngas, a guard bed may be used. Especially to remove all traces of HCN and/or NH₃ specific types of active coal may be used. Trace amounts of sulphur may be removed by an absorption process using iron and/or zinc oxide.

The purified gaseous mixture, comprising predominantly hydrogen, carbon monoxide and optionally nitrogen, is contacted with a suitable catalyst in the catalytic conversion stage, in which the normally liquid hydrocarbons are formed. Suitably at least 70 v/v% of the syngas is contacted with the catalyst, preferably at least 80%, more preferably at least 90, still more preferably all the syngas.

The catalysts used for the catalytic conversion of the mixture comprising hydrogen and carbon monoxide into hydrocarbons are known in the art and are usually referred to as Fischer-Tropsch catalysts. Catalysts for use in the Fischer-Tropsch hydrocarbon synthesis process frequently comprise, as the catalytically active component, a metal from Group VIII of the Periodic Table of Elements. Particular catalytically active metals include ruthenium, iron, cobalt and nickel. Cobalt is a preferred catalytically active metal.

The catalytically active metal is preferably supported on a porous carrier. The porous carrier may be selected from any of the suitable refractory metal oxides or silicates or combinations thereof known in the art. Particular examples of preferred porous carriers include silica, alumina, titania, zirconia, ceria, gallia and mixtures thereof, especially silica and titania.

The amount of catalytically active metal on the carrier is preferably in the range of from 3 to 300 pbw per 100 pbw of carrier material, more preferably from 10 to 80 pbw, especially from 20 to 60 pbw.

If desired, the catalyst may also comprise one or more metals or metal oxides as promoters. Suitable metal oxide promoters may be selected from Groups IIA, IIIB, IVB, VB and VIB of the Periodic Table of Elements, or the actinides and lanthanides. In particular, oxides of magnesium, calcium, strontium, barium, scandium, yttrium. lanthanum, cerium, titanium, zirconium, hafnium, thorium, uranium, vanadium, chromium and manganese are most suitable promoters. Particularly preferred metal oxide promoters for the catalyst used to prepare the waxes for use in the present invention are manganese and zirconium oxide. Suitable metal promoters may be selected from Groups VIIB or VIII of the Periodic Table. Rhenium and Group VIII noble metals are particularly suitable, with platinum and palladium being especially preferred. The amount of promoter present in the catalyst is suitably in the range of from 0.01 to 100 pbw, preferably 0.1 to 40, more preferably 1 to 20 pbw, per 100 pbw of carrier.

The catalytically active metal and the promoter, if present, may be deposited on the carrier material by any suitable treatment, such as impregnation, kneading and extrusion. After deposition of the metal and, if appropriate, the promoter on the carrier material, the loaded carrier is typically subjected to calcination at a temperature of generally from 350 to 750 °C, preferably a temperature in the range of from 450 to 550 °C. The effect of the calcination treatment is to remove crystal water, to decompose volatile decomposition products and to convert organic and inorganic compounds to their respective oxides. After calcination, the resulting catalyst may be activated by contacting the catalyst with hydrogen or a hydrogen-containing gas, typically at temperatures of about 200 to 350 °C.

The catalytic conversion process may be performed under conventional synthesis conditions known in the art. Typically, the catalytic conversion may be effected at a temperature in the range of from 100 to 450 °C, preferably from 150 to 350 °C, more preferably from 180 to 270 °C. Typical total pressures for the catalytic conversion process are in the range of from 1 to 200 bar absolute, more preferably from 10 to 70 bar absolute. In the catalytic conversion process especially (more than 50 wt% of C₅+, preferably 70 wt%) C₅₋₂₀ hydrocarbons are formed.

Preferably, a Fischer-Tropsch catalyst is used, which yields substantial quantities of paraffins, more preferably substantially unbranched paraffins. A part may boil above the boiling point range of the so-called middle distillates, but it might be desired to keep this part relatively small to avoid problems with respect to normally solid hydrocarbons. A most suitable catalyst for this purpose is a cobalt-containing Fischer-Tropsch catalyst. The term "middle distillates", as used herein, is a reference to hydrocarbon mixtures of which the boiling point range corresponds substantially to that of kerosine and gas oil fractions obtained in a conventional atmospheric distillation of crude mineral oil. The boiling point range of middle distillates generally lies within the range of about 150 to about 360 °C.

The higher boiling range paraffinic hydrocarbons, if present, may be isolated and subjected to a catalytic hydrocracking, which is known per se in the art, to yield the desired middle distillates. The catalytic hydro-cracking is carried out by contacting the paraffinic hydrocarbons at elevated temperature and pressure and in the presence of hydrogen with a catalyst containing one or more metals having hydrogenation activity, and supported on a carrier. Suitable hydrocracking catalysts include catalysts comprising metals selected from Groups VIB and VIII of the Periodic Table of Elements. Preferably, the hydrocracking catalysts contain one or more noble metals from group VIII. Preferred noble metals are platinum, palladium, rhodium, ruthenium, iridium and osmium. Most preferred catalysts for use in the hydro-cracking stage are those comprising platinum. To keep the process as simple as possible, the hydrocracking will usually not be a preferred option.

The amount of catalytically active metal present in the hydrocracking catalyst may vary within wide limits and is typically in the range of from about 0.05 to about 5 parts by weight per 100 parts by weight of the carrier material.

Suitable conditions for the catalytic hydrocracking are known in the art. Typically, the hydrocracking is effected at a temperature in the range of from about 175 to 400 °C. Typical hydrogen partial pressures applied in the hydrocracking process are in the range of from 10 to 250 bar.

The process may conveniently and advantageously be operated in a single pass mode ("once through") devoid of any recycle streams, thus allowing the process to be comparatively simple and relatively low cost. The process may be carried out in one or more reactors, either parallel or in series. In the case of small hydro-carbonaceous feedstock streams, the preference will be to use only one reactor. Slurry bed reactors, ebulliating bed reactors and fixed bed reactors may be used, the fixed bed reactor being the preferred option.

The product of the hydrocarbon synthesis suitably comprises mainly normally liquid hydrocarbons, beside water and gaseous hydrocarbons. By selecting the catalyst and the process conditions in such a way that especially normally liquid hydrocarbons are obtained, the product obtained ("syncrude") may transported in the liquid form or be mixed with any stream of crude oil without creating any problems as to solidification and or crystallisation of the mixture. It is observed in this respect that the production of heavy hydrocarbons, comprising large amounts of solid wax, are less suitable for mixing with crude oil while transport in the liquid form has to be done at elevated temperatures, which is less desired.

The off gas of the hydrocarbon synthesis comprises normally gaseous hydrocarbons produced in the synthesis process, nitrogen, unconverted methane and other feed stock hydrocarbons, unconverted carbon monoxide and hydrogen, carbon dioxide and water. The normally gaseous hydrocarbons are suitably C₁₋₅ hydrocarbons, preferably C₁₋₄ hydrocarbons, more preferably C₁₋₃ hydrocarbons. These hydrocarbons, or mixtures thereof, are gaseous at temperatures of 5-30 °C (1 bar), especially at 20 °C (1 bar). Further, oxygenated compounds, e.g. methanol, dimethylether, may be present in the off gas. The off gas is to be utilised for the production of electrical power, in an expanding/combustion process. The energy generated in the process may be used for own use or for export to local customers. At least part of the energy is used for the compression of the hydrocarbonaceous feed. Further, it might be used for the compression of the oxygen containing gas and the oxygen enrichment of the gas, especially by membrane techniques. Suitably at least 70 v/v% of the off gas is used for power generation, preferably at least 80%, more preferably at least 90, still more preferably all the off gas.

An attractive method for generating power is a combined gas turbine/steam turbine cycle. In short, the principle of operation of the gas turbine/stem turbine system is that the remaining gaseous mixture is first combusted, the combustion gases are then expanded in the gas turbine, thereby generating power, and the hot exhaust gas from the gas turbine is thereafter used in the production of high pressure steam. The steam thus generated is expanded in a steam turbine and produces an additional quantity of power. Turbines of this kind are known in the art. In a preferred embodiment the process water of the hydrocarbon synthesis step is added to the feed of the expanding/combustion process.

To increase the carbon efficiency of the process, carbon monoxide may be separated from hydrocarbon synthesis off gas. This can be done by techniques known in the art, for example by membrane separation techniques or by pressure swing absorption. By recirculation of the carbon monoxide stream to the CPO unit and/or to hydro-carbon synthesis unit, an increased conversion of starting hydrocarbonaceous feed into normally liquid hydrocarbons is obtained. Further, less energy (power) is produced which will be advantageous in remote area's with no direct use for the power generated. Further, an additional stream is obtained which can be used to tune the H₂/CO ratio. To increase the carbon efficiency of the process even further, also carbon dioxide may be separated from the off gas, and recirculated to the syngas preparation step.

In a further embodiment of the invention, hydrogen may be separated from the synthesis gas obtained in the first step. The hydrogen is preferably separated after quenching/cooling, and may be separated by techniques well known in the art, as pressure swing adsorption, or, preferably, by means of membrane separation techniques. The hydrogen may be used in a second heavy paraffin synthesis step after the first reactor (provided that a two stage hydrocarbon synthesis is used), or for other purposes, e.g. hydrotreating and/or hydrocracking of hydrocarbons produced in the paraffin synthesis. In this way a further product optimisation is obtained (for instance by fine tuning the H₂/CO ratio's in the first and second hydrocarbon synthesis step), while also the carbon efficiency can be improved. In addition, the product quality may be improved by e.g. hydrogenation and/or hydrocracking.

The product of the hydrocarbon synthesis reaction of step (ii) may contain a certain amount of heavy wax, depending on the catalyst and the reaction conditions. The amount of heavy wax may be up to 40 wt%, sometimes up to 60 wt% and sometimes even up to 90 wt%.

Any percentage mentioned in this description is calculated on total weight or volume of the composition, unless indicated differently. When not mentioned, percentages are considered to be weight percentages. Pressures are indicated in bar absolute, unless indicated differently.

## Claims

1. Process for producing normally liquid hydrocarbons from a hydrocarbonaceous feed which process comprises the following steps:
(i) partial oxidation of the hydrocarbonaceous feed at elevated temperature and pressure using an oxygen containing gas to obtain synthesis gas;
(ii) catalytically converting at least part of the synthesis gas mixture obtained in step (i) at elevated temperature and pressure into normally liquid hydrocarbons, normally gaseous hydrocarbons, optionally heavy wax and water;
(iii) expanding and/or combusting at least part of the normally gaseous hydrocarbons produced in step (ii) to provide power for compressing the hydrocarbonaceous feed used in step (i), the gaseous hydrocarbons containing any unconverted synthesis gas and/or hydrocarbonaceous feed.

2. Process according to claim 1, in which the hydro-carbonaceous feed is methane, natural gas, associated gas or a mixture of C₁₋₄ hydrocarbons, preferably associated gas.

3. Process according to claim 2, in which the associated gas is associated gas at a remote location or at an offshore platform.

4. Process according to any of claims 1 to 3, in which the oxygen containing gas is a pressurised oxygen containing gas, preferably obtained by compressing the oxygen containing gas with power generated by expanding and/or combusting at least part of the normally gaseous hydrocarbons produced in step (ii).

5. Process according to any of claims 1 or 4, in which the partial oxidation is a catalytic partial oxidation, preferably, preferably carried out at a temperature between 800 and 1200 °C more preferably 850 and 1050 °C, and at a pressure between 10 and 50 bar, more preferably between 20 and 40 bar.

6. Process according to any of claims 1 to 5, in which the oxygen containing gas is air, or oxygen enriched air, preferably containing up to 60 volume percent oxygen (on total), more preferably up to 40 volume percent.

7. Process according to claim 6, in which the oxygen enriched air is made by a membrane based process, preferably produced by using power obtained by expanding and/or combusting at least part of the normally gaseous hydrocarbons produced in step (ii).

8. Process according to any of claims 1 to 7, in which the partial oxidation is carried out in the presence of steam and/or water, preferably made in the catalytic hydrocarbon synthesis reaction, and/or carbon dioxide.

9. Process according to any of claims 1 to 8, in which mainly (at least 50 wt% of C₅+, preferably 70 wt%) C₅₋₂₀ hydrocarbons are formed step (ii).

10. Process according to any of claims 1 to 9, in which step (ii) is carried out in a once through process.

11. Process according to any of claims 1 to 10, in which process water obtained in step (ii) (preferably at least 50, more preferably 70%) is used in the expanding/combustion process of step (iii).

12. Process according to any of claims 1 to 11, in which part of the energy made in step (iii) is converted into electricity, is used for desalination and/or is used for reinjection associated gas into underground formations.

13. Process according to any of claims 1 to 12, in which the process is carried out on a barge or floating vessel or on an off shore platform.
